# EUROPEAN PATENT SPECIFICATION

(11) **EP 2 325 156 B1**
(45) Date of publication and mention of the grant of the patent: **10.10.2018**
(21) Application number: 09810068.8
(22) Date of filing: 24.08.2009
(51) Int. Cl.: C07C 1/207, C07C 11/02, C07B 61/00

(54) **PROCESS FOR PRODUCTION OF OLEFIN**
VERFAHREN ZUR HERSTELLUNG VON OLEFINEN
PROCÉDÉ DE PRODUCTION D OLÉFINE

(30) Priority: 25.08.2008 JP 2008214859; 19.12.2008 JP 2008324120; 19.12.2008 JP 2008324223; 24.12.2008 JP 2008327404; 12.03.2009 JP 2009058970
(43) Date of publication of application: 25.05.2011
(73) Proprietor: Kao Corporation, Chuo-ku Tokyo 103-8210 (JP)
(72) Inventor: SUZUKI, Nobuyoshi, Wakayama-shi Wakayama 640-8580 (JP); TAHARA, Hideo, Wakayama-shi Wakayama 640-8580 (JP); ISHIHARA, Daisuke, Wakayama-shi Wakayama 640-8580 (JP); DANJO, Hiroshi, Wakayama-shi Wakayama 640-8580 (JP); MIMURA, Taku, Wakayama-shi Wakayama 640-8580 (JP); FUKUYAMA, Takahide, 1-1, Gakuencho, Naka-ku Sakai-shi, Osaka 5998531 (JP); RYU, Ilhyong, 1-1, Gakuencho, Naka-ku Sakai-shi, Osaka 5998531 (JP)
(74) Representative: Hoffmann Eitle
(86) International application number: PCT/JP2009/065131
(87) International publication number: WO 2010/024420

(56) References cited:
- JP-A- 50 014 603
- JP-A- 50 047 904
- JP-A- 50 116 403
- US-A- 3 530 198
- US-A- 3 530 198
- US-A- 3 625 996
- US-A- 4 554 397
- US-A- 5 077 447
- US-A- 5 077 447
- MILLER J A ET AL: "A HIGHLY CATALYTIC AND SELECTIVE CONVERSION OF CARBOXYLIC ACIDS TO 1-ALKENES OF ONE LESS CARBON ATOM", THE JOURNAL OF ORGANIC CHEMISTRY, AMERICAN CHEMICAL SOCIETY, US, vol. 58, no. 1, January 1993 (1993-01), pages 18-20, XP000325380, ISSN: 0022-3263, DOI: 10.1021/JO00053A008
- GOOSSEN LUKAS J ET AL: "A mild and efficient protocol for the conversion of carboxylic acids to olefins by a catalytic decarbonylative elimination reaction", CHEMICAL COMMUNICATIONS > CHEMICAL COMMUNICATIONS - CHEMCOM; [6015D], ROYAL SOCIETY OF CHEMISTRY, GB, no. 6, 21 March 2004 (2004-03-21), pages 724-725, XP002636756, ISSN: 1359-7345, DOI: 10.1039/B316613A [retrieved on 2004-02-18]

## Description

### Field of the invention

The present invention relates to a process for producing an olefin from a carboxylic acid having a β-hydrogen atom or a derivative thereof, and particularly to a process for producing an olefin suitably used as an intermediate material for surfactants, various chemicals, and medicines.

### Background of the invention

To produce an olefin having an intended chain length, generally known is a method of oligomerizing a short-chain olefin such as ethylene to give an α-olefin. The method of oligomerizing however produces olefins having different polymerization degrees and cannot selectively provide an olefin having an intended chain length at high yield.

There are also known methods for producing an olefin from a carboxylic acid, including that of producing an olefin from a carboxylic acid in the presence of a Pd complex catalyst (US-A3530198), that of producing an α-olefin from a carboxylic acid in the presence of an acid anhydride and a catalyst containing an element selected from Groups 8, 9, and 10 metals and copper (US-A5077447), and that of producing an α-olefin from a carboxylic acid using a Pd complex catalyst and pivalic anhydride (Chem. Commun., 724, (2004)). These methods employ a special additive and/or a high reaction temperature of 250°C or more in order to effectively obtain olefin, but resulting in unsatisfactory yields of desired olefins.

US 3,625,996 concerns a process for preparation of olefinic acids or esters by contacting a saturated dicarboxylic acid or ester thereof at elevated temperature with a complex catalyst comprising a Group VIII noble metal and a ligand from the group consisting of organic phosphines, arsines and stibines.

### Summary of the Invention

The present invention provides a decarbonylation process for producing an olefin from a carboxylic acid having a β-hydrogen atom or a derivative thereof, using a compound containing iodine and at least one metal element selected from the group consisting of metals of Groups 8, 9 and 10 as a catalyst; wherein the carboxylic acid having a β-hydrogen atom or a derivative thereof is a monocarboxylic acid having a β-hydrogen atom or an anhydride thereof, a halide thereof, an ester thereof or an amide thereof (hereinafter referred to as embodiment 1).

The present invention also provides a process for producing an olefin, comprising decarbonylation of a monocarboxylic acid having a β-hydrogen atom or an anhydride thereof in the presence of an iodide and a catalyst comprising an element selected from metals of Groups 8, 9, and 10, the amount of the catalyst being 0.00001 to 0.2 mol based on the metal atom to one mole of the carboxylic acid having the β-hydrogen atom, and the amount of the iodide being 0.001 to 10 mol based on the iodine atom to on mole of the carboxylic acid having the β-hydrogen atom. (hereinafter referred to as embodiment 2).

### Detailed description of the invention

The present invention relates to provide a process for producing an olefin, that enables to produce an intended olefin at high yield.

According to the process of the present invention, an olefin suitably used as a base for surfactants or an intermediate material for various compounds can be synthesized at high yield from a carboxylic acid or a derivative thereof.

### [Embodiment 1]

In the embodiment 1 of the present invention, the carboxylic acid having a β-hydrogen atom or a derivative thereof is a monocarboxylic acid having a β-hydrogen atom or an anhydride thereof, a halide thereof, an ester thereof or an amide thereof. The carboxylic acid may be saturated, unsaturated, partially cyclic, or heteroatom-containing, or may have a plurality of carbonyl groups. Preferred are saturated monocarboxylic acids and derivatives thereof. Examples of the carboxylic acid derivative having a β-hydrogen atom include, carboxylic anhydrides, carboxylic acid halides, carboxylic acid esters, and carboxylic acid amides, all which have a β-hydrogen atom. Preferred are carboxylic anhydrides having a β-hydrogen atom and carboxylic acid halides having a β-hydrogen atom, and more preferred are carboxylic anhydrides having a β-hydrogen atom.

Specific examples of the carboxylic acid having a β-hydrogen atom include caproic acid, caprylic acid, capric acid, lauric acid, myristic acid, palmitic acid, stearic acid, behenic acid, 3-phenylpropionic acid, eicosanoic acid, 9-decenoic acid, 10-undecenoic acid, oleic acid, 2,4-hexadienoic acid, 3-methylbutanoic acid, 6-octadecynoic acid, hydnocarpic acid, gorlic acid, and ricinoleic acid.

Specific examples of the carboxylic anhydride having a β-hydrogen atom include caproic anhydride, caprylic anhydride, capric anhydride, lauric anhydride, myristic anhydride, palmitic anhydride, stearic anhydride, behenic anhydride, 3-phenylpropionic anhydride, eicosanoic anhydride, 9-decenoic anhydride, 10-undecenoic anhydride, oleic anhydride, 2,4-hexadienoic anhydride, 3-methylbutanoic anhydride, 6-octadecynoic anhydride, hydnocarpic anhydride, gorlic anhydride, ricinoleic anhydride and succinic anhydride. Specific examples also include condensed carboxylic anhydrides produced from carboxylic acids described for above specific examples of the carboxylic acid with different carboxylic acids for above specific examples or acids such as formic acid, acetic acid, propionic acid, and butyric acid.

Specific examples of the carboxylic acid halide having a β-hydrogen atom include chlorides, bromides, and iodides of caproic acid, caprylic acid, capric acid, lauric acid, myristic acid, palmitic acid, stearic acid, behenic acid, 3-phenylpropionic acid, eicosanoic acid, 9-decenoic acid, 10-undecenoic acid, oleic acid, 2,4-hexadienoic acid, 3-methylbutanoic acid, 6-octadecynoic acid, hydnocarpic acid, gorlic acid or ricinoleic acid.

Specific examples of the carboxylic acid ester having a β-hydrogen atom include methyl ester and ethyl ester of caproic acid, caprylic acid, capric acid, lauric acid, myristic acid, palmitic acid, stearic acid, behenic acid, 3-phenylpropionic acid, eicosanoic acid, 9-decenoic acid, 10-undecenoic acid, oleic acid, 2,4-hexadienoic acid, 3-methylbutanoic acid, 6-octadecynoic acid, hydnocarpic acid, gorlic acid, or ricinoleic acid.

Specific examples of the carboxylic acid amide having a β-hydrogen atom include amides, monomethylamides, dimethylamides, and diethylamides of caproic acid, caprylic acid, capric acid, lauric acid, myristic acid, palmitic acid, stearic acid, behenic acid, 3-phenylpropionic acid, eicosanoic acid, 9-decenoic acid, 10-undecenoic acid, oleic acid, 2,4-hexadienoic acid, 3-methylbutanoic acid, 6-octadecynoic acid, hydnocarpic acid, gorlic acid or ricinoleic acid.

In the carboxylic acid having a β-hydrogen atom or a derivative thereof, the number of carbon atoms of the carboxylic acid or a carboxylic acid residue (in cases of carboxylic anhydrides, the number of carbon atoms of at least one carboxylic acid residue) is preferably 3 to 22, more preferably 8 to 18, and even more preferably: 12 to 18. It is noted that an unsaturated carboxylic acid or a derivative thereof produces an olefin having one additional double bond to the double bond(s) of the starting acid or derivative.

In the embodiment 1 of the present invention, the catalyst used is a compound containing iodine and at least one metal element selected from the group consisting of Groups 8, 9 and 10 metals. Examples of the metal element include Fe, Ru, Co, Rh, Ir, Ni, Pd, and Pt. Among these metal elements, preferred are Groups 9 and 10 metals, and more preferred is a Group 9 metal selected from Co, Rh, and Ir.

Specific examples of the compound containing iodine and at least one metal element selected from the group consisting of Groups 8, 9 and 10 metals used as the catalyst in the embodiment 1 of the present invention include: FeI₂, FeI₃, FeI(CO)₂(C₅H₅), RuI₃, RuI₂(CO)₂, RuI(CO)₂(C₅H₅), CoI₂, CoI₂(CO)(C₅H₅), CoI₂(PPh₃)(C₅H₅), CoI₂(PPh₃)₂, RhI₃, [RhI(CO)₂]₂, RhI(PPh₃)₃, IrI₄, IrI(CO)(PPh₃)₂, NiI₂, NiI₂(NH₃)₆, NiI(1,5-cyclooctadiene), NiI(PPh₃)₃, PdI₂, PdI₂(PPh₃)₂, PdI(CH₃)(PPh₃)₂, PtI₂, [Pt₂I₂(H₂NCH₂CH₂NH₂)₂](NO₃)₂, PtI₂(1,5-cyclooctadiene), PtI(CH₃)₃, and PtI(CH₃)(PEt₃)₂ (wherein, Ph represents phenyl group, and Et represents ethyl group, the same meanings are applied below) . Preferred are CoI₂, RhI₃, [RhI(CO)₂]₂, IrI(CO)(PPh₃)₂, NiI₂, FeI₂, and PtI₂, and more preferred are CoI₂, RhI₃, [RhI(CO)₂]₂, IrI(CO)(PPh₃)₂, and NiI₂.

These catalysts may also be used together with a ligand, including N-heterocyclic carbene ligands, pyridine ligands, such as 2,2-bipyridyl and pyridine, arsenic ligands, nitrile ligands, such as acetonitrile and benzonitrile, isonitrile ligands, and organophosphorus ligands. When used, preferred are organophosphorus ligands. Examples of the organophosphorus ligand include dimethylphenylphosphine, diethylphenylphosphine, methyldiphenylphosphine, ethyldiphenylphosphine, cyclohexyldiphenylphosphine, tricyclohexylphosphine, triisopropylphosphine, tributylphosphine, tri-t-butylphosphine, tribenzylphosphine, triphenylphosphine, tris(para-methoxyphenyl)phosphine, and 1,2-bis(diphenylphosphino)ethane. Preferred are triphenylphosphine and 1,2-bis(diphenylphosphino)ethane. These ligands may be used alone or in combination.

In the embodiment 1 of the present invention, a used amount of the catalyst is preferably 0.00001 to 0.2 mol, more preferably 0.0001 to 0.05 mol, even more preferably 0.001 to 0.04 mol, even more preferably 0.005 to 0.03 mol, as the metal atom, to one mole of the carboxylic acid having a β-hydrogen atom or a derivative thereof.

In the embodiment 1 of the present invention, the reaction will progress with or without an acid anhydride. When the acid anhydride is added, an additive amount of the acid anhydride is preferably not more than 10 mol, and more preferably not more than 2 mol to one mole of the carboxylic acid having a β-hydrogen atom or a derivative thereof. The additive amount of the acid anhydride is also preferably not less than 0.01 mol. Preferred are acetic anhydride, propionic anhydride, and pivalic anhydride, and more preferred is acetic anhydride.

In the embodiment 1 of the present invention, from the viewpoint of achieving good selectivity in olefin production, a reaction temperature of decarbonylation is preferably 20 to 300°C, more preferably 80 to 270°C, and even more preferably 120 to 260°C.

### [Embodiment 2]

In the embodiment 2 of the present invention, for the carboxylic acid having a β-hydrogen atom or an anhydride thereof, those described in the embodiment 1 is used. In the carboxylic acid having a β-hydrogen atom or a derivative thereof of the embodiment 2, the number of carbon atoms of the carboxylic acid or a carboxylic acid residue (in cases of carboxylic anhydrides, at least one carboxylic acid residue) is preferably 3 to 22, and more preferably 3 to 18.

Hereinafter, an embodiment using an carboxylic acid having a β-hydrogen atom as the carboxylic acid having a β-hydrogen atom or an anhydride thereof will be described (hereinafter, referred to as embodiment 2(1)).

In the embodiment 2(1) of the present invention, the catalyst used contains an element selected from Groups 8, 9, and 10 metals. From the viewpoints of reactivity and selectivity, the catalyst preferably contains an element of Group 9 metal or Group 10 metal. Examples of the element of Group 8, 9, and 10 metals include Fe, Co, Ni, Ru, Rh, Pd, Os, Ir, and Pt. Preferred are Co, Ni, Rh, Pd, and Ir, more preferred are Co, Rh, Pd, and Ir, and even more preferred is Rh. Specific examples of the catalyst containing an element selected from Groups 8, 9, and 10 metals and copper include [RhCl(CO)₂]₂, (Ph₃P)₂Rh(CO)Cl, (Ph₃P)₂NiCl₂, (Ph₃P)₂PdCl₂, (Ph₃P)₂CoCl₂, (Ph₃P)₂PtCl₂, (Ph₃P)₂Ir(CO)Cl, (Ph₃P)₃CuCl (wherein, Ph represents a phenyl group, the same is applied below). Preferred are [RhCl(CO)₂]₂, (Ph₃P)₂PdCl₂, (Ph₃P)₂CoCl₂, (Ph₃P)₂Ir(CO)Cl, and (Ph₃P)₂NiCl₂.

These catalysts may further preferably be used together with a ligand, including N-heterocyclic carbene ligands, pyridine ligands such as 2,2-bipyridyl and pyridine, arsenic ligands, nitrile ligands such as acetonitrile and benzonitrile , isonitrile ligands, and organophosphorus ligands. When used, preferred are organophosphorus ligands. Specific examples of the organophosphorus ligand include those described for the embodiment 1. Among them, preferred for the embodiment 2(1) are triphenylphosphine, dimethylphenylphosphine, diethylphenylphosphine, methyldiphenylphosphine, ethyldiphenylphosphine, and 1,2-bis(diphenylphosphino ethane). These ligands may be used alone or in combination.

From the viewpoint of achieving good stability of the catalyst and reaction rate, a used amount of the ligand is preferably in the range from 0.1 to 1000 mol, more preferably 0.2 to 500 mol, and even more preferably 0.3 to 100 mol of ligand molecule including those that the catalyst originally has to one mole of metal atom of a Group 8 metal, Group 9 metal, or Group 10 metal compound, based on an element selected from Groups 8, 9, and 10 metals.

In the embodiment 2 (1) of the present invention, an used amount of the catalyst containing an element selected from Groups 8, 9, and 10 metals is 0.00001 to 0.2 mol, more preferably 0.0001 to 0.05 mol, even more preferably 0.001 to 0.03 mol, and still even more preferably 0.005 to 0.025 mol based on the metal atom to one mole of the carboxylic acid having a β-hydrogen atom.

In the embodiment 2(1) of the present invention, any iodide can be used without specific limitation. Examples of the iodide include iodides of Groups 1 to 14 elements and a quaternary ammonium compound represented by formula (1).

[R-(Y)ₙ]₄N⁺I⁻ (1)

wherein, R represents a hydrocarbon group having 1 to 22 carbon atoms; Y represents a group -Z-(CH₂)ₘ-, wherein Z represents an ether, amino, amide or ester group, more particularly -O-, -NH-, -CONH-, -NHCO-, -COO- or -OCO-, and m represents the number of 1 to 6; n represents 0 or 1; plural R's, plural Y's, and plural n's may be the same as or different from one another; and two [R-(Y)ₙ]s may together form a cyclic structure.

Any iodide of Groups 1 to 14 elements can be used without specific limitation. Preferred are iodides of Groups 1, 11, and 12 elements. Specific examples of the iodide include KI, ·CuI, LiI, NaI, and ZnI₂. Preferred are KI and NaI.

The quaternary ammonium compound represented by formula (1) is preferably a compound in which R represents an alkyl group having 1 to 7 carbon atoms or a benzyl group (preferably an alkyl group having 1 to 7 carbon atoms), and n represents 0. More preferred are Et₄N⁺I⁻, (n-Butyl)₄N⁺I⁻ (wherein, Et represents an ethyl group, and n-Butyl represents an n-butyl group), and even more preferred is Et₄N⁺I⁻.

In the embodiment 2(1) of the present invention, a used amount of the iodide is 0.001 to 10 mol and even more preferably 0 . 01 to 3 mol to one mole of the carboxylic acid having a β-hydrogen atom.

In the embodiment 2(1) of the present invention, the reaction will progress without an acid anhydride, but more progress even at low temperature by adding an acid anhydride. In this case, an additive amount of the acid anhydride is preferably not more than 10 mol, and more preferably not more than 2 mol to one mole of the carboxylic acid having a β-hydrogen atom. The additive amount of the acid anhydride is also preferably not less than 0.01 mol. Preferred acid anhydrides are acetic anhydride, propionic anhydride, and pivalic anhydride, and more preferred is acetic anhydride.

From the viewpoint of achieving good selectivity in olefin production, in the embodiment 2(1) of the present invention, a reaction temperature of decarbonylation is preferably 20 to 300°C, more preferably 80 to 280°C, and even more preferably 130 to 260°C.

Next, an embodiment using a carboxylic anhydride having a β-hydrogen atom as the carboxylic acid having a β-hydrogen atom or a derivative thereof will be described (hereinafter, referred to as embodiment 2(2)).

In the embodiment 2 (2) of the present invention, for the carboxylic anhydride having a β-hydrogen atom, those described in the embodiment 1 can be used. Examples of the carboxylic anhydride include a compound represented by formula (2): 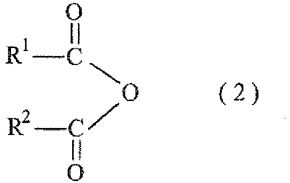 wherein, R¹ represents an optionally substituted hydrocarbon group having a hydrogen atom on the β-position of a carbonyl group; and R² represents a hydrogen atom or an optionally substituted hydrocarbon group.

In formula (2), R¹ preferably represents an alkyl or alkenyl group having 2 to 21 carbon atoms and having a hydrogen atom on the β-position of a carbonyl group, and more preferably an alkyl group. The number of carbon atoms is preferably 3 to 17, and more preferably 11 to 17. R² preferably represents an alkyl or alkenyl group having 1 to 21 carbon atoms, and more preferably the same group to R¹. R¹ and R² may also together form a cyclic group. Examples of the substituent on a hydrocarbon group include a hydroxy group, an alkoxy group, and a halogen atom.

Specific examples of the carboxylic anhydride having a β-hydrogen atom used in the embodiment 2(2) of the present invention include those described in the embodiment 1. Among them, preferred are caproic anhydride, caprylic anhydride, capric anhydride, lauric anhydride, myristic anhydride, palmitic anhydride, stearic anhydride, eicosanoic anhydride, behenic anhydride, and oleic anhydride, and more preferred is stearic anhydride.

For producing the carboxylic anhydride having a β-hydrogen atom used in the present invention, any method can be used without specific limitation. Examples of the method include that of dehydration of a carboxylic acid with an agent such as thionyl chloride, phosphoryl chloride, acetic anhydride, trifluoroacetic anhydride, and acetyl chloride, that of reaction of a carboxylic acid halide with a carboxylic acid alkaline metal or alkaline earth metal salt, and that of oxidation of an aldehyde. Preferred are methods of reaction of a carboxylic acid halide with a carboxylic acid alkaline metal or alkaline earth metal salt and of dehydration of a carboxylic acid with acetic anhydride.

In the embodiment 2(2) of the present invention, similar to the embodiment 2(1), the catalyst used contains an element selected from Groups 8, 9, and 10 metals. In the embodiment 2(2), preferred elements of Groups 8, 9, and 10 metals are Fe, Co, Ni, Ru, Rh, Pd, Ir, and Pt, more preferred are Ni, Ru, Rh, Pd, and Ir, and even more preferred are Ni, Rh, Pd, and Ir. Specific examples of the catalyst used in the embodiment 2(2) include [RhCl(CO)₂]₂, (Ph₃P)₂Rh(CO)Cl, (Ph₃P)₃RhCl, (Ph₃P)₂NiCl₂, (Ph₃P)₂PdCl₂, (Ph₃P)₄Pd, Pd(OAc)₂, (Ph₃P)₂CoCl₂, CoCl₂, (Ph₃P)₂PtCl₂, FeCl₂, Ru₃(CO)₁₂, [RuCl₂(CO)₃]₂, (Ph₃P)₃RuCl₂, (Ph₃P)₄RuCl₂, (Ph₃P)₂Ir(CO)Cl and IrCl(CO)₃, (wherein, Ph represents a phenyl group, the same is applied below). Preferred are [RhCl(CO)₂]₂, (Ph₃P)₂PdCl₂, IrCl(CO)₃, (Ph₃P)₂NiCl₂, Ru₃(CO)₁₂, CoCl₂, and FeCl₂, and more preferred are [RhCl(CO)₂]₂, (Ph₃P)₂PdCl₂, IrCl(CO)₃, (Ph₃P)₂NiCl₂, and Ru₃(CO)₁₂.

These catalysts, similar to embodiments 1 and 2(1), may also be used together with a ligand, including N-heterocyclic carbene ligands, pyridine ligands such as 2,2-bipyridyl and pyridine, arsenic ligands, nitrile ligands such as acetonitrile and benzonitrile, isonitrile ligands, and organophosphorus ligands. Specific examples of the organophosphorus ligand include those described in the embodiment 1. These ligands may be used alone or in combination. A used amount of the ligand is as described in the embodiment 2(1).

In the embodiment 2(2), a used amount of the catalyst containing an element selected from Groups 8, 9, and 10 metals is 0.00001 to 0.2 mol, more preferably 0.0001 to 0.05 mol, even more preferably 0.001 to 0.03 mol, and still even more preferably 0.005 to 0.025 mol as the metal atom to one mole of the carboxylic anhydride having a β-hydrogen atom.

In the embodiment 2(2) of the present invention, the iodide used is as described in the embodiment 2(1).

In the embodiment 2(2) of the present invention, a used amount of the iodide is 0.001 to 10 mol, and more preferably 0.01 to 2 mol based on the iodine atom to one mole of the carboxylic anhydride having a β-hydrogen atom.

From the viewpoint of achieving good selectivity in olefin production, in the embodiment 2(2) of the present invention, a reaction temperature of decarbonylation is preferably 20 to 300°C, more preferably 80 to 250°C, and even more preferably 120 to 220°C.

The olefin produced according to the process of the embodiment 1, 2(1), or 2(2) of the present invention may be a terminal olefin having a double bond at the terminal in structure or an internal olefin having a double bond shifted to an inner position isomerized from the terminal olefin.

The olefin produced according to the process of the present invention can be suitably used as an intermediate material for surfactants, various chemicals, and medicines.

### Examples

The following Examples demonstrate the present invention.

In the following description, unless otherwise specified, "%" refers "% by mol". Examples are assigned as follows: "Example 1-n" for Example according to embodiment 1 and "Example 2-n" and "Example 3-n" for Examples according to embodiments 2(1) and 2(2), respectively.

### Example 1-1

In a screw-top test tube with a septum, under nitrogen atmosphere, 142.2 mg of stearic acid (0.5 mmol), 1.6 mg of CoI₂ (0.005 mmol), 2.0 mg of 1,2-bis(diphenylphosphino)ethane (0.005 mmol), and 51.0 mg of acetic anhydride (0.5 mmol) were stirred with a stirring bar for 3 hours at 250°C. Then having stopped heating, the mixture was allowed to cool to a room temperature (25°C). Low boiling-point components were distilled off under reduced pressure. To the product was added 30.3 mg of anisole as an internal standard. The product was measured by ¹H-NMR to quantify the starting material and produced materials. The quantification of the starting material and produced materials was conducted by comparing integrated intensities of peaks of α-proton of stearic acid, vinyl proton of a terminal olefin, vinyl proton of an internal olefin, and methyl group proton of anisole as the internal standard.

A conversion rate of stearic acid was 56%, and yields of a terminal olefin and an internal olefin were 43% and 5%, respectively, to the starting stearic acid.

### Comparative Example 1-1

This Example was carried out in the same way as Example 1-1, except that CoCl₂ was used instead of CoI₂.

A conversion rate of stearic acid was 68%. A terminal olefin and an internal olefin were not produced.

### Example 1-2

In a screw-top test tube with a septum, under nitrogen atmosphere, 142.2 mg of stearic acid (0.5 mmol) and 8.7 mg of IrI(CO)(PPh₃)₂ (0.01 mmol) were stirred with a stirring bar for 3 hours at 250°C. Then stopped heating, the mixture was allowed to cool to a room temperature (25°C) . To the product was added 30.3 mg of anisole as an internal standard. In the same way as in Example 1-1, the product was measured by ¹H-NMR to quantify the starting material and produced materials.

A conversion rate of stearic acid was 87% and a yield of an internal olefin was 87% to the stearic acid used.

### Comparative Example 1-2

This Example was carried out in the same way as Example1-2, except that IrCl(CO)(PPh₃)₂ was used instead of IrI(CO)(PPh₃)₂.

A conversion rate of stearic acid was 68%. The yield of a terminal olefin was 3% and the yield of an internal olefin was 65%, to the starting stearic acid.

Results of Examples 1-1 and 1-2 and Comparative Examples 1-1 and 1-2 are summarized in Table 1.

**Table 1**

| | Example 1-1 | Comparative example 1-1 | Example 1-2 | Comparative example 1-2 |
|---|---|---|---|---|
| Kind of catalyst | CoI₂ | CoCl₂ | IrI(CO)(PPh₃)₂ | IrCl(CO)(PPh₃)₂ |
| Amount of catalyst(% by mole to stearic acid) | 1 | 1 | 2 | 2 |
| Additive amount of 1,2-bis(diphenylphosphino) ethane | 1 | 1 | - | - |
| (% by mole to stearic acid) | | | | |
| Additive amount of acetic anhydride | 100 | 100 | - | - |
| (% by mole to stearic acid) | | | | |
| Conversion rate (%) | 56 | 68 | 87 | 68 |
| Total yield of olefin(% by mole) | 48 | - | 87 | 68 |
| Yield of internal olefin (% by mole) | 5 | - | 87 | 65 |
| Yield of terminal olefin(% by mole) | 43 | - | - | 3 |

### Example 1-3

In a 50 mL recovery flask, placed were a stirring bar, 12.4 g of stearic anhydride (22.5 mmol), and 377 mg of [RhI(CO)₂]₂ (0.66 mmol) . The inside atmosphere of the flask was substituted with nitrogen. With keeping a pressure to 0.02 MPa, the mixture were stirred for 6 hours at 160°C. Then stopped heating, to the product was added 50 mg of anisole as an internal standard. In the same way as in Example 1-1, the product was measured by ¹H-NMR to quantify the starting material and produced materials.

A conversion rate of stearic anhydride was 100%, and the yields of a terminal olefin, an internal olefin, and stearic acid were 19%, 80%, and 99%, respectively, to the starting stearic anhydride.

### Comparative Examples 1-3 and 1-4

These Examples were carried out in the same way as Example 1-3, except that catalysts shown in Table 2 were used instead of [RhI(CO)₂]₂.

Results of Example 1-3 and Comparative Examples 1-3 and 1-4 are summarized in Table 2.

**Table 2**

| | Example | Comparative example | |
|---|---|---|---|
| | 1-3 | 1-3 | 1-4 |
| Kind of catalyst | [RhI(CO)₂]₂ | [RhCl(CO)₂]₂ | [RhBr(CO)₂]₂ |
| Amount of catalyst | 3 | 3 | 3 |
| (% by mole to stearic anhydride) | | | |
| Conversion rate (%) | 100 | 3 | 27 |
| Total yield of olefin(% by mote) | 99 | 3 | 17 |
| Yield of internal olefin (% by mole) | 80 | 3 | 12 |
| Yield of terminal olefin (% by mole) | 19 | 0 | 5 |
| Yield of stearic acid(% by mole) | 99 | 3 | 22 |

### Example 1-4

In a 50 mL recovery flask, a stirring bar, 4.1 g of stearic anhydride (7.5 mmol), 72.5 mg of RhI₃ (0.15 mmol), and 157 mg of PPh₃ (0.60 mmol) were introduced. The inside atmosphere of the flask was substituted with nitrogen. With keeping a pressure to 0.033 MPa, the mixture was stirred for 3 hours at 200°C. Then stopped heating, to the product was added 50 mg of anisole as an internal standard. In the same way as in Example 1-1, the product was measured by ¹H-NMR to quantify the starting material and produced materials.

A conversion rate of stearic anhydride was 100%, and the yields of a terminal olefin, an internal olefin, and stearic acid were 7%, 88%, and 99%, respectively, to the starting stearic anhydride.

### Examples 1-5 to 1-7 and Comparative Examples 1-5 to 1-8

These Examples were carried out in the same way as Example 1-4, except that catalysts and reaction temperatures shown in Table 3 were used.

Results of Examples 1-4 to 1-7 and Comparative Examples 1-5 to 1-8 are summarized in Table 3.

**Table 3**

| | Example | | | | Comparative example | | | |
|---|---|---|---|---|---|---|---|---|
| | 1-4 | 1-5 | 1-6 | 1-7 | 1-5 | 1-6 | 1-7 | 1-8 |
| Kind of catalyst | RhI₃ | NiI₂ | FeI₂ | PtI₂ | RhCl₃ | NiCl₂ | FeCl₂ | PtCl₂ |
| Amount of catalyst | 2 | 2 | 2 | 2 | 2 | 2 | 2 | 2 |
| (% by mole to stearic anhydride) | | | | | | | | |
| Adding amount of PPh₃ | 8 | 8 | 8 | 8 | 8 | 8 | 8 | 8 |
| by mole to stearic anhydride) | | | | | | | | |
| Reaction temperature (°C) | 200 | 200 | 250 | 250 | 200 | 200 | 250 | 250 |
| Conversion rate (%) | 100 | 100 | 32 | 38 | 0 | 0 | 24 | 28 |
| Total yield of olefins (% by mole) | 95 | 100 | 16 | 21 | - | - | 2 | 13 |
| Yield of internal olefin (% by mole) | 88 | 92 | 11 | 14 | - | - | 1 | 4 |
| Yield of terminal olefin (% by mole) | 7 | 8 | 5 | 7 | - | - | 1 | 9 |
| Yield of stearic acid (% by mole) | 99 | 100 | 32 | 38 | - | - | 24 | 28 |

### Example 1-8

In a 50 mL recovery flask, a stirring bar, 4.3 g of stearic acid (15 mmol), 72.5 mg of RhI₃ (0.15 mmol), and 157 mg of PPh₃ (0.60 mmol) were introduced. The inside atmosphere of the flask was substituted with nitrogen. With keeping a pressure to 0.033 MPa, the mixture was stirred for 3 hours at 250°C. Then stopped heating, to the product was added 50 mg of anisole as an internal standard. In the same way as in Example 1-1, the product was measured by ¹H-NMR to quantify the starting material and produced materials.

A conversion rate of stearic acid was 24%, and the yields of a terminal olefin and an internal olefin were 2% and 22%, respectively, to the starting stearic acid.

### Examples 1-9 to 1-11 and Comparative Examples 1-9 to 1-12

These Examples were carried out in the same way as Example1-8, except that catalysts shown in Table 4 were used instead of RhI₃.

Results of Examples 1-8 to 1-11 and Comparative Examples 1-9 to 1-12 are summarized in Table 4.

**Table 4**

| | Example | | | | Comparative example | | | |
|---|---|---|---|---|---|---|---|---|
| | 1-8 | 1-9 | 1-10 | 1-11 | 1-9 | 1-10 | 1-11 | 1-12 |
| Kind of catalyst | RhI₃ | NiI₂ | FeI₂ | PdI₂ | RhCl₃ | NiCl₂ | FeCl₂ | PdCl₂ |
| Conversion rate (%) | 24 | 20 | 2 | 14 | 20 | 10 | 0 | 9 |
| Total yield of olefins (% by mole) | 24 | 8 | 2 | 10 | 16 | 1 | - | 7 |
| Yield of internal olefin (% by mole) | 22 | 7 | 2 | 10 | 15 | 1 | - | 5 |
| Yield of terminal olefin(% by mole) | 2 | 1 | 0 | 0 | 1 | 0 | - | 2 |

### Example 2-1

In a 10 mL recovery flask, 568.7 mg of stearic acid (2.0 mmol), 7.8 mg of rhodium catalyst [RhCl(CO)₂]₂ (0.02 mmol), 31.8 mg of 1,2-bis(diphenylphosphino)ethane (0.08 mmol), and 332 mg of potassium iodide (2.0 mmol) were stirred with a stirring bar at 250°C. Immediately after starting to heat, the solid of stearic acid melted to become a uniform solution, and the solution began to bubble. After three hours, stopped heating, the mixture was allowed to cool to a room temperature and filtered while washing with ethyl ether. Ethyl ether was distilled off under reduced pressure. To the product was added 105.9 mg of anisole as an internal standard. Similarly as in Example 1-1, the product was measured by ¹H-NMR to quantify the starting material and produced materials.

A result of gas chromatography (GC) measurement showed a small amount of starting stearic acid remaining. A result of ¹H-NMR measurement showed that an amount of the starting stearic acid remaining was 13%, and the amounts of a terminal olefin and an internal olefin were 10% and 60%, respectively.

### Example 2-2 and Comparative Example 2-1

These Examples were carried out in the same way as Example 2-1, except that potassium iodide was used in an amount as shown in Table 5.

### Example 2-3

The Example was similarly performed as Example 2-1, except that lauric acid was used instead of stearic acid.

Results of Examples 2-1 to 2-3 and Comparative Example 2-1 are summarized in Table 5.

**Table 5**

| | Catalyst | Iodide | | Additive amount of acetic anhydride | Reaction temperature (°C) | Reaction time (h) | Result of ¹H-NMR quantification of product (%) | | | Conversion rate (%) |
|---|---|---|---|---|---|---|---|---|---|---|
| | | Kind | Adding amount (molar multiplies ^{*1}) | | | | Starting material | Internal olefin | Terminal olefin | |
| Example 2-1 | [RhCl(CO)₂]₂ | KI | 1 | Not added | 250 | 3 | 13 | 60 | 10 | 87 |
| Example 2-2 | [RhCl(CO)₂]₂ | KI | 2 | Not added | 250 | 5 | 8 | 65 | 16 | 92 |
| Example 2-3 | [RhCl(CO)2]2 | KI | 1 | Not added | 250 | 3 | 18 | 41 | 41 | 82 |
| Comparative example 2-1 | [RhCl(CO)₂]₂ | None | - | Not added | 250 | 3 | 50 | 35 | 8 | 50 |

| | | | | | | | | | | |
|---|---|---|---|---|---|---|---|---|---|---|
| *1 : Amount to 1 mole of fatty acid | | | | | | | | | | |

### Example 2-4

In a 10 mL recovery flask, 568.7 mg of stearic acid (2.0 mmol), 7.8 mg of rhodium catalyst [RhCl(CO)₂]₂ (0.02 mmol), 31.8 mg of 1,2-bis(diphenylphosphino)ethane (0.08 mmol), 56.9 mg of acetic anhydride (0.2 mmol), 332 mg of potassium iodide (2.0 mmol) were stirred with a stirring bar at 250°C. Immediately after starting to heat, the solid of stearic acid melted to give a uniform solution, and the solution began to bubble. After three hours, stopped heating, the mixture was allowed to cool to a room temperature (25°C) and filtered while washing with ethyl ether. Ethyl ether was distilled off under reduced pressure. To the product was added 105.9 mg of anisole as an internal standard. Similarly as in Example 1-1, the product was measured by ¹H-NMR to quantify the starting material and produced materials.

A result of GC measurement showed a small amount of starting stearic acid remaining. A result of ¹H-NMR measurement showed that an amount of the starting stearic acid remaining was 4%, and the amounts of a terminal olefin and an internal olefin were 8% and 86%, respectively.

### Example 2-5

The Example was similarly performed as Example 2-4, except that tetraethylammonium iodide was used instead of potassium iodide.

A result of GC measurement showed a small amount of starting stearic acid remaining. A result of ¹H-NMR measurement showed that an amount of the starting stearic acid remaining was 12%, and the amounts of a terminal olefin and an internal olefin were 1% and 63%, respectively.

### Comparative Example 2-2

The Example was carried out in the same way as Example 2-4, except that potassium iodide was not used.

A result of ¹H-NMR measurement showed that an amount of the starting stearic acid remaining was 38%, and the amounts of a terminal olefin and an internal olefin were 6% and 45%, respectively.

Results of Examples 2-4 and 2-5 and Comparative Example 2-2 are summarized in Table 6.

**Table 6**

| | Catalyst | Iodide | | Additive amount of acetic anhydride (Molar multiplies ^{*1}) | Reaction temperature (°C) | Reaction time Starting (h) | Result of 1H-NMR quantification of product (%) | | | Conversion rate (%) |
|---|---|---|---|---|---|---|---|---|---|---|
| | | Kind | Additive amount (Molar multiplies ^{*1}) | | | | material | Internal olefin | Terminal olefin | |
| Example 2-4 | [RhCl(CO)₂]₂ | KI | 1 | 0.1 | 250 | 3 | 4 | 86 | 8 | 96 |
| Example 2-5 | [RhCl(CO)₂]₂ | Et₄NI | 1 | 0.1 | 250 | 3 | 12 | 63 | 1 | 88 |
| Comparative example 2-2 | [RhCl(CO)₂]₂ | None | - | 0.1 | 250 | 3 | 38 | 45 | 6 | 62 |

| | | | | | | | | | | |
|---|---|---|---|---|---|---|---|---|---|---|
| *1 : Amount to 1 mole of stearic acid | | | | | | | | | | |

### Example 2-6

The Example was carried out in the same way as Example 2-4, except that an amount of acetic anhydride and a reaction temperature were changed as shown in Table 7.

A result of GC measurement showed a small amount of starting stearic acid remaining. A result of ¹H-NMR measurement showed that an amount of the starting stearic acid remaining was 6%, and the amounts of a terminal olefin and an internal olefin were 65% and 38%, respectively.

### Comparative Example 2-3

In a 10 mL recovery flask, 568.7 mg of stearic acid (2.0 mmol), 7.8 mg of rhodium catalyst [RhCl(CO)₂]₂ (0.02 mmol), 31.8 mg of 1,2-bis(diphenylphosphino)ethane (0.08 mmol), 569 mg of acetic anhydride (2 mmol) were stirred with a stirring bar at 140°C. Immediately after starting to heat, the solid of stearic acid melted to give a uniform solution, and the solution began to bubble. After three hours, stopped heating, the mixture was allowed to cool to a room temperature (25°C) and filtered, while washing with ethyl ether. To the product was added 95.9 mg of n-nonadecane as an internal standard. The product was measured by GC measurement. A result showed that there was almost no olefin produced.

Results of Example 2-6 and Comparative Example 2-3 are summarized in Table 7.

**Table 7**

| | Catalyst | Iodide | | Additive amount of acetic anhydride (Molar multiplies *¹) | Reaction temperature (°C) | Reaction time (h) | Result of 1H-NMR quantification of product (%) | | | Conversion rate (%) |
|---|---|---|---|---|---|---|---|---|---|---|
| | | Kind | Additive amount (Molar multiplies *¹) | | | | Starting material | Internal olefin | Terminal olefin | |
| Example 2-6 | [RhCl(CO)₂]₂ | KI | 1 | 1 | 140 | 3 | 6 | 38 | 65 | 94 |
| Comparative example 2-3 | (RhCl(CO)₂]₂ | None | - | 1 | 140 | 3 | 100 | - | - | 0 |

| | | | | | | | | | | |
|---|---|---|---|---|---|---|---|---|---|---|
| *1 : Amount to 1 mole of stearic acid | | | | | | | | | | |

### Example 2-7

In a 50 mL recovery flask, 13.0607 g of stearic acid (0.046 mol), 0.316 g of palladium catalyst [(Ph₃P)₂PdCl₂] (0. 00045 mol), 5.9 g of triphenylphosphine (Ph₃P) (0.0225 mol), 2.49 g of potassium iodide (0.015 mol), and 0.9581 g of squalane as an internal standard were stirred with a stirring bar for 24 hours at 250°C. A sample was then taken and subjected to a GC analysis . A result of GC analysis showed that a yield of olefin was 90.6%.

### Comparative Example 2-4

In a 50 mL recovery flask, 12.7069 g of stearic acid (0.045 mol), 0.316 g of palladium catalyst [(Ph₃P)₂PdCl₂] (0.00045 mol), 2.36 g of triphenylphosphine (Ph₃P) (0.0135 mol), and 0.9684 g of squalane as an internal standard were stirred with a stirring bar for 24 hours at 250°C. A sample was then taken and subjected to a GC analysis. A result of GC analysis showed that a yield of olefin was 16.2%.

Results of Example 2-7 and Comparative Example 2-4 are summarized in Table 8.

**Table 8**

| | Catalyst | Amount of Ph₃P ligand (molar multiplies *²) | Iodide | | Reaction temperature (°C) | Reaction time (h) | Yield of olefin (%) |
|---|---|---|---|---|---|---|---|
| | | | Kind | Additive amount (molar multiplies *¹) | | | |
| Example 2-7 | [(Ph₃P)₂PdCl₂] | 50 | KI | 0.33 | 250 | 24 | 90.6 |
| Comparative example 2-4 | [(Ph₃P)₂PdCl₂] | 30 | None | - | 250 | 24 | 16.2 |

| | | | | | | | |
|---|---|---|---|---|---|---|---|
| *1 : Amount to 1 mole of stearic acid *2: Amount of 1 mole of catalyst | | | | | | | |

### Example 2-8

In a screw-top test tube with a septum, 142.2 mg of stearic acid (0.5 mmol), 3.3 mg of cobalt catalyst [(Ph₃P)₂CoCl₂] (0.005 mmol), 51.0 mg of acetic anhydride (0.5 mmol), and 83.0 mg of potassium iodide (0.5 mmol) were stirred with a stirring bar for 3 hours at 250°C. Then stopped heating, the mixture was allowed to cool to a room temperature (25°C) and filtered, while washing with ethyl ether. Ethyl ether was distilled off under reduced pressure. To the product was added anisole as an internal standard. The product was measured by ¹H-NMR.

A result of ¹H-NMR measurement showed that an amount of the starting stearic acid remaining was 22%, and the amounts of a terminal olefin and an internal olefin were 16% and 11%, respectively.

### Comparative Example 2-5

The Example was carried out in the same way as Example 2-8, except that potassium iodide was not used.

A result of ¹H-NMR measurement showed that an amount of the starting stearic acid remaining was 2%, and there was no olefin.

Results of Example 2-8 and Comparative Example 2-5 are summarized in Table 9.

**Table 9**

| | Catalyst | Iodide | | Additive amount of acetic anhydride (molar multiplies*¹) | Reaction temperature (°C) | Reaction time (h) | Result of ¹H-NMR quantification of product (%) | | | Conversion rate (%) |
|---|---|---|---|---|---|---|---|---|---|---|
| | | Kind | Additive amount (molar multiplies *¹) | | | | Starting material | Internal olefin | Terminal olefin | |
| Example 2-8 | [(Ph₃P)₂CoCl₂] | KI | 1 | 1 | 250 | 3 | 22 | 11 | 16 | 78 |
| Comparative example 2-5 | [(Ph₃P)₂CoCl₂] | None | - | 1 | 250 | 3 | 2 | - | - | 98 |

| | | | | | | | | | | |
|---|---|---|---|---|---|---|---|---|---|---|
| *1 : Amount to 1 mole of stearic acid | | | | | | | | | | |

### Example 2-9

In a screw-top test tube with a septum, 142 .2 mg of stearic acid (0.5 mmol), 7.8 mg of iridium catalyst [(Ph₃P)₂Ir(CO)Cl] (0.01 mmol), and 75.0 mg of sodium iodide (0.5 mmol) were stirred with a stirring bar for 3 hours at 250°C. Then stopped heating, the mixture was allowed to cool to a room temperature (25°C) and filtered, while washing with ethyl ether. Ethyl ether was distilled off under reduced pressure. To the product was added anisole as an internal standard. The product was measured by ¹H-NMR.

A result of ¹H-NMR measurement showed that an amount of the starting stearic acid remaining was 19%, an amount of an internal olefin was 81%, and there was no terminal olefin.

### Comparative Example 2-6

The Example was carried out in the same way as Example 2-9, except that sodium iodide was not used.

A result of ¹H-NMR measurement showed that an amount of the starting stearic acid remaining was 32%, and the amounts of a terminal olefin and an internal olefin were 3% and 65%, respectively.

Results of Example 2-9 and Comparative Example 2-6 are summarized in Table 10.

**Table 10**

| | Catalyst | Iodide | | Reaction temperature (°C) | Reaction time (h) | Result of 1H-NMR quantification of product ) | | | Conversion rate ) |
|---|---|---|---|---|---|---|---|---|---|
| | | Kind | Adding amount (molar multiplies *¹⁾ | | | Satarting material | Internal olefin | Terminal olefin | |
| Example 2-9 | [(Ph₃P)₂Ir(CO)Cl] | NaI | 1 | 250 | 3 | 19 | 81 | - | 81 |
| Comparative example 2-6 | [(Ph₃P)₂Ir(CO)Cl] | None | - | 250 | 3 | 32 | 65 | 3 | 68 |

| | | | | | | | | | |
|---|---|---|---|---|---|---|---|---|---|
| *1:Amount to 1 mole of stearic acid | | | | | | | | | |

### Example 2-10

In a 20 mL recovery flask, 1.28 g of stearic acid (4.5 mmol), 58.8 mg of nickel catalyst [(Ph₃P)₂NiCl₂] (0.09 mmol), 47.2 mg of triphenylphosphine (Ph₃P) (0.18 mmol), 1.49 g of potassium iodide (9 mmol) were stirred with a stirring bar for 3 hours at 250°C. To the product was added anisole as an internal standard. The product was measured by ¹H-NMR.

A result of ¹H-NMR measurement showed that an amount of the starting stearic acid remaining was 74%, and the amounts of a terminal olefin and an internal olefin were 1% and 11%, respectively.

### Comparative Example 2-7

The Example was similarly performed as Example 2-10, except that potassium iodide was not used.

A result of ¹H-NMR measurement showed that an amount of the starting stearic acid remaining was 87%, an amount of an internal olefin was 4%, and there was no terminal olefin.

Results of Example 2-10 and Comparative Example 2-7 are summarized in Table 11.

**Table 11**

| | Catalyst | Iodide | | Reaction temperature (°C) | Reaction time (h) | Result of 1H-NMR quantification of product (%) | | | Conversion rate (%) |
|---|---|---|---|---|---|---|---|---|---|
| | | Kind | Additive amount (molar multiplies^{*1}) | | | Starting material | Internal olefin | Terminal olefin | |
| Example 2-10 | [(Ph₃P)₂NiCl₂] | KI | 2 | 250 | 3 | 74 | 11 | 1 | 26 |
| Comparative example 2-7 | [(Ph₃P)₂NiCl₂] | None | - | 250 | 3 | 87 | 4 | - | 13 |

| | | | | | | | | | |
|---|---|---|---|---|---|---|---|---|---|
| *1 : Amount to 1 mole of stearic acid | | | | | | | | | |

### Preparation Example 3-1

In a 500 mL reaction vessel, to 154 g of stearic acid chloride (0.51 mol) was added 153 g of sodium stearate (0.50 mol) over 30 minutes at 25°C with stirring. The mixture was then stirred for 2 hours at 90°C. The reaction mixture was filtered and crystallized by adding petroleum ether. The resultant crystals were filtered, washed, and dried under reduced pressure to give 198 g of stearic anhydride (0.36 mol) .

### Preparation Example 3-2

In a 300 mL reaction vessel, a stirring bar, 142 g of stearic acid (0.50 mol) and 51 g of acetic anhydride (0.50 mol) were heated to 120°C at 27 kPa. The inside pressure of the reaction system was decreased to 1.3 kPa over 2 hours. Generating acetic acid was distilled off. The mixture was further reacted for 4 hours at 150°C (1.3 kPa) to give 137 g of stearic anhydride (0.50 mol) .

### Example 3-1

In a 50 mL recovery flask, placed were 12.4 g of stearic anhydride (22.5 mmol) prepared according to Preparation Example 1, 316 mg of (Ph₃P)₂PdCl₂ (0.45 mmol), 236 mg of PPh₃ (0.90 mmol), and 2.5 g of potassium iodide (15 mmol). The inside atmosphere of the flask was substituted with nitrogen. With keeping a pressure to 0.033 MPa, the mixture were stirred with a stirring bar for 3 hours at 160°C. Then stopped heating, to the product was added anisole as an internal standard. In the same way as Example 1-1, the product was measured by ¹H-NMR to quantify the starting material and produced materials.

A conversion rate of stearic anhydride was 68%. The yields of a terminal olefin, an internal olefin and stearic acid were 24%, 42%, and 68%, respectively, to the starting stearic anhydride.

### Comparative Example 3-1

The Example was carried out in the same way as Example 3-1, except that potassium iodide was not used.

A conversion rate of stearic anhydride was 6%, and the yields of a terminal olefin and stearic acid were 3% and 5%, respectively, to the starting stearic anhydride.

### Examples 3-2 to 3-7

These Examples were carried out in the same way as Example 3-1, except that PPh₃ and potassium iodide were used in amounts as shown in Table 12.

Results of Examples 3-1 to 3-7 and Comparative Example 3-1 are summarized in Table 12.

**Table 12**

| | Example | | | | | | | Comparative example |
|---|---|---|---|---|---|---|---|---|
| | 3-1 | 3-2 | 3-3 | 3-4 | 3-5 | 3-6 | 3-7 | 3-1 |
| Kind of catalyst | (Ph₃P)₂PdCl₂ | | | | | | | |
| Amount of catalyst | 2 | 2 | 2 | 2 | 2 | 2 | 2 | 2 |
| (% by mole to stearic anhydride) | | | | | | | | |
| Additive amount of PPh3 | 4 | 6 | 8 | 20 | 4 | 4 | 4 | 4 |
| (% by mole to stearic anhydride) | | | | | | | | |
| Additive amount of KI{% by mole to stearic anhydride) | 67 | 67 | 67 | 67 | 4 | 20 | 40 | - |
| Conversion rate (%) | 68 | 63 | 40 | 13 | 49 | 71 | 73 | 6 |
| Total yield of olefin (% by mole) | 66 | 61 | 39 | 9 | 48 | 71 | 73 | 3 |
| Yield of internal olefin (% by mole) | 42 | 30 | 18 | 3 | 24 | 36 | 42 | 0 |
| Yield of terminal olefin (% by mole) | 24 | 31 | 21 | 6 | 24 | 35 | 31 | 3 |
| Yield of stearic acid(% by mole) | 68 | 63 | 40 | 13 | 49 | 70 | 73 | 5 |

### Examples 3-8 to 3-10 and Comparative Example 3-2

These Examples were carried out in the same way as Example 3 - 1, except that kinds and amounts of the catalyst and the iodide used were as shown in Table 13 and PPh₃ was not added.

Results of Examples 3-8 to 3-10 and Comparative Example 3-2 are summarized in Table 13.

**Table 13**

| | Example | | | Comparative example |
|---|---|---|---|---|
| | 3-8 | 3-9 | 3-10 | 3-2 |
| Kind of catalyst | [RhCl(CO)₂]₂ | [RhCl(CO)₂]₂ | [RhCl(CO)₂]₂ | [RhCl(CO)₂]₂ |
| Amount of catalyst | 0.67 | 0.67 | 0.67 | 0.67 |
| (% by mole to stearic anhydride) | | | | |
| Kind of iodide | KI | NaI | (n-butyl)₄NI | - |
| Additive amount of iodide (% by moleto stearic anhydride) | 67 | 67 | 67 | - |
| Conversion rate (%) | 99 | 99 | 23 | 0 |
| Total yield of olefin (% by mole) | 99 | 99 | 17 | 0 |
| Yield of internal olefin (% by mole) | 78 | 97 | 10 | 0 |
| Yield of terminal olefin (% by mole) | 21 | 2 | 7 | 0 |
| Yield of stearic acid (% by mole) | 99 | 99 | 13 | 0 |

### Examples 3-11 to 3-16 and Comparative Examples 3-3 to 3-8

These Examples were similarly performed as Example 3-1, except that a type of catalyst, amounts of catalyst, PPh₃, and potassium iodide, and a reaction temperature used were as shown in Table 14 and stearic anhydride prepared in Preparation Example 3-2 was used.

Results of Examples 3-11 to 3-16 and Comparative Examples 3-3 to 3-8 are summarized in Table 14.

**Table 14**

| | Example | | | | | | Comparative example | | | | | |
|---|---|---|---|---|---|---|---|---|---|---|---|---|
| | 3-11 | 3-12 | 3-13 | 3-14 | 3-15 | 3-16 | 3-3 | 3-4 | 3-5 | 3-6 | 3-7 | 3-8 |
| Kind of catalyst | [RhCl(CO)₂]₂ | IrCl(CO)₃ | (Ph₃P)₂NiCl₂ | Ru₃(CO)₁₂ | CoCl₂ | FeCl₂ | [RhCl(CO)₂]₂ | IrCl(CO)₃ | (Ph₃P)_{z}NiCl₂ | Ru₃(CO)₁₂ | CoCl₂ | FeCl₂ |
| Amount of catalyst | 1 | 2 | 2 | 0.67 | 2 | 2 | 1 | 2 | 2 | 0.67 | 2 | 2 |
| (% by mole to stearic anhydride) | | | | | | | | | | | | |
| Additive amount of PPh₃ | - | - | - | - | - | 8 | - | - | - | - | - | 8 |
| (% by moe to stearic anhydride) | | | | | | | | | | | | |
| Additive amount of KI | 20 | 20 | 20 | 20 | 20 | 20 | - | - | - | - | - | - |
| (% by mole to stearic anhydride) | | | | | | | | | | | | |
| Reaction temperature | 160°C | 160°C | 200°C | 200°C | 250°C | 250°C | 160°C | 160°C | 200°C | 200°C | 250°C | 250°C |
| Conversion rate (%) | 100 | 98 | 74 | 30 | 26 | 56 | 0 | 54 | 0 | 15 | 0 | 24 |
| Total yield of oledin(% by mole) | 99 | 98 | 74 | 20 | 7 | 10 | 0 | 38 | 0 | 11 | 0 | 2 |
| Yield of internal olefin (% by mole) | 72 | 98 | 43 | 20 | 7 | 7 | 0 | 14 | 0 | 11 | 0 | 1 |
| Yield of terminal olefin (% by mole) | 27 | 0 | 31 | 0 | 0 | 3 | 0 | 24 | 0 | 0 | 0 | 1 |
| Yield of stearic acid(% by mole) | 100 | 98 | 74 | 29 | 21 | 10 | 0 | 46 | 0 | 15 | 0 | 24 |

## Claims

1. A decarbonylation process for producing an olefin from a carboxylic acid having a β-hydrogen atom or a derivative thereof, using a compound containing iodine and at least one metal element selected from the group consisting of metals of Groups 8, 9 and 10 as a catalyst; wherein the carboxylic acid having a β-hydrogen atom or a derivative thereof is a monocarboxylic acid having a β-hydrogen atom or an anhydride thereof, a halide thereof, an ester thereof or an amide thereof.

2. The process for producing an olefin according to claim 1, wherein the monocarboxylic acid having a β-hydrogen atom or a derivative thereof is a monocarboxylic acid having a β-hydrogen atom or a monocarboxylic anhydride having a β-hydrogen atom.

3. The process for producing an olefin according to claim 1 or 2, wherein the compound comprising iodine and at least one metal element selected from the group consisting of metals of Groups 8, 9 and 10 is a compound comprising iodine and at least one metal element selected from the group consisting of metals of Groups 9 and 10.

4. A process for producing an olefin, comprising decarbonylation of a monocarboxylic acid having a β-hydrogen atom or an anhydride thereof in the presence of an iodide and a catalyst comprising an element selected from metals of Groups 8, 9, and 10, the amount of the catalyst being 0.00001 to 0.2 mol based on the metal atom to one mole of the carboxylic acid having the β-hydrogen atom, and the amount of the iodide being 0.001 to 10 mol based on the iodine atom to one mole of the carboxylic acid having the β-hydrogen atom.

5. The process for producing an olefin according to claim 4, wherein the element selected from the group consisting of metals of Groups 8, 9, and 10 is an element selected from the group consisting of Groups 9 and 10.

6. The process for producing an olefin according to claim 4 or 5, wherein the iodide is an iodide of an element selected from the group consisting of metals of Groups 1 to 14 or a quaternary ammonium compound represented by formula (1):
[R-(Y)ₙ]₄N⁺I⁻ (1)
wherein, R represents a hydrocarbon group having 1 to 22 carbon atoms; Y represents a group -Z-(CH₂)ₘ-, wherein Z represents an ether, amino, amide or ester group, and m represents the number of 1 to 6; n represents 0 or 1;
R's, Y's, and n's may be the same as or different from one another; and two [R-(Y)ₙ]S may together form a cyclic structure.

## Patentansprüche

1. Decarbonylierungsverfahren zur Herstellung eines Olefins aus einer Carbonsäure mit einem β-Wasserstoffatom oder einem Derivat davon unter Verwendung einer Verbindung, enthaltend Iod und mindestens ein Metallelement, ausgewählt aus der Gruppe, bestehend aus Metallen der Gruppen 8, 9 und 10, als Katalysator; wobei die Carbonsäure mit einem β-Wasserstoffatom oder ein Derivat davon eine Monocarbonsäure mit einem β-Wasserstoffatom oder ein Anhydrid davon, ein Halogenid davon, ein Ester davon oder ein Amid davon ist.

2. Verfahren zur Herstellung eines Olefins gemäß Anspruch 1, wobei die Monocarbonsäure mit einem β-Wasserstoffatom oder ein Derivat davon eine Monocarbonsäure mit einem β-Wasserstoffatom oder ein Monocarbonsäureanhydrid mit einem β-Wasserstoffatom ist.

3. Verfahren zur Herstellung eines Olefins gemäß Anspruch 1 oder 2, wobei die Verbindung, die Iod und mindestens ein Metallelement, ausgewählt aus der Gruppe, bestehend aus Metallen der Gruppen 8, 9 und 10, umfasst, eine Verbindung ist, die Iod und mindestens ein Metallelement, ausgewählt aus der Gruppe, bestehend aus Metallen der Gruppen 9 und 10, umfasst.

4. Verfahren zur Herstellung eines Olefins, umfassend die Decarbonylierung einer Monocarbonsäure mit einem β-Wasserstoffatom oder eines Anhydrids davon in Gegenwart eines Iodids und eines Katalysators, umfassend ein Element, ausgewählt aus Metallen der Gruppen 8, 9 und 10, wobei die Menge des Katalysators 0,00001 bis 0,2 Mol, bezogen auf das Metallatom, auf ein Mol der Carbonsäure mit dem β-Wasserstoffatom beträgt und die Menge des Iodids 0,001 bis 10 Mol, bezogen auf das Iodatom, auf ein Mol der Carbonsäure mit dem β-Wasserstoffatom beträgt.

5. Verfahren zur Herstellung eines Olefins gemäß Anspruch 4, wobei das Element, ausgewählt aus der Gruppe, bestehend aus Metallen der Gruppen 8, 9 und 10, ein Element ist, das aus der Gruppe, bestehend aus den Gruppen 9 und 10, ausgewählt ist.

6. Verfahren zur Herstellung eines Olefins gemäß Anspruch 4 oder 5, wobei das Iodid ein Iodid eines Elements, ausgewählt aus der Gruppe, bestehend aus Metallen der Gruppen 1 bis 14, oder eine quartäre Ammoniumverbindung, dargestellt durch Formel (1), ist:
[R-(Y)ₙ]₄N⁺I⁻ (1)
worin R für eine Kohlenwasserstoffgruppe mit 1 bis 22 Kohlenstoffatomen steht; Y für eine Gruppe -Z-(CH₂)ₘ-steht, worin Z für eine Ether-, Amino-, Amid- oder Estergruppe steht und m für eine Zahl von 1 bis 6 steht; n für 0 oder 1 steht;
die R's, die Y's und die n's gleich oder voneinander verschieden sein können; und zwei [R-(Y)ₙ]S zusammen eine cyclische Struktur bilden können.

## Revendications

1. Procédé de décarbonylation pour produire une oléfine à partir d'un acide carboxylique ayant un atome d'hydrogène en β ou d'un dérivé de celui-ci, en utilisant un composé contenant de l'iode et au moins un élément métallique sélectionné dans le groupe constitué des métaux des groupes 8, 9 et 10 comme catalyseur; dans lequel l'acide carboxylique ayant un atome d'hydrogène en β ou un dérivé de celui-ci est un acide monocarboxylique ayant un atome d'hydrogène en β ou un anhydride de celui-ci, un halogénure de celui-ci, un ester de celui-ci ou un amide de celui-ci.

2. Procédé pour produire une oléfine selon la revendication 1, dans lequel l'acide monocarboxylique ayant un atome d'hydrogène en β ou un dérivé de celui-ci est un acide monocarboxylique ayant un atome d'hydrogène en β ou un anhydride monocarboxylique ayant un atome d'hydrogène en β.

3. Procédé pour produire une oléfine selon la revendication 1 ou 2, dans lequel le composé comprenant de l'iode et au moins un élément métallique sélectionné dans le groupe constitué des métaux des groupes 8, 9 et 10 est un composé comprenant de l'iode et au moins un élément métallique sélectionné dans le groupe constitué des métaux des groupes 9 et 10.

4. Procédé pour produire une oléfine, comprenant la décarbonylation d'un acide monocarboxylique ayant un atome d'hydrogène en β ou d'un anhydride de celui-ci en présence d'iodure et d'un catalyseur comprenant un élément sélectionné parmi les métaux des groupes 8, 9 et 10, la quantité du catalyseur étant de 0,00001 à 0,2 mole sur la base de l'atome métallique pour une mole de l'acide carboxylique ayant l'atome d'hydrogène en β, et la quantité de l'iodure étant de 0,001 à 10 moles sur la base de l'atome d'iode pour une mole de l'acide carboxylique ayant l'atome d'hydrogène en β.

5. Procédé pour produire une oléfine selon la revendication 4, dans lequel l'élément sélectionné dans le groupe constitué des métaux des groupes 8, 9 et 10 est un élément sélectionné dans le groupe constitué des groupes 9 et 10.

6. Procédé pour produire une oléfine selon la revendication 4 ou 5, dans lequel l'iodure est un iodure d'un élément sélectionné dans le groupe constitué des métaux des groupes 1 à 14 ou d'un composé d'ammonium quaternaire représenté par la formule (1) :
[R-(Y)ₙ]₄N⁺I⁻ (1)
dans laquelle, R représente un groupe hydrocarboné contenant 1 à 22 atomes de carbone ; Y représente un groupe -Z-(CH₂)ₘ-, dans lequel Z représente un groupe éther, amino, amide ou ester, et m représente un nombre allant de 1 à 6 ; n représente 0 ou 1 ;
les R, les Y, et les n peuvent être identiques les uns aux autres ou différents les uns des autres ; et deux [R-(Y)ₙ] peuvent former ensemble une structure cyclique.
